(19) Europäisches Patentamt
European Patent Office
Office européen des brevets

(11) Publication number: **0 260 880 A2**

# (12) EUROPEAN PATENT APPLICATION

(21) Application number: **87308016.2**

(22) Date of filing: **10.09.87**

(51) Int. Cl.4: **A61K 35/12** , A61K 39/395 , C12N 15/00 , C12P 21/00

The microorganism(s) has (have) been deposited with American Type Culture Collection under number(s) ATCC 40268.

(30) Priority: **11.09.86 US 906413**
**18.11.86 US 932871**

(43) Date of publication of application:
**23.03.88 Bulletin 88/12**

(84) Designated Contracting States:
**BE DE FR GB IT NL SE**

(71) Applicant: **DANA-FARBER CANCER INSTITUTE, INC.**
**44 Binney Street**
**Boston Massachusetts MA 02115(US)**

(72) Inventor: **Reinherz, Ellis L.**
**113 South Great Road**
**Lincoln Massachusetts 01773(US)**
Inventor: **Siliciano, Robert F.**
**99 Marion Street**
**Brookline Massachusetts 02146(US)**
Inventor: **Sayre, Peter**
**Two Peabody Terrace Apt. 406**
**Cambridge Massachusetts 02138(US)**
Inventor: **Chang, Hsiu-Ching**
**16 Lowell Street**
**Cambridge Massachusetts 02138(US)**
Inventor: **Richardson, Neil**
**367 Hunnewell Street**
**Needham Heights Massachusetts 02194(US)**

(74) Representative: **Field, Roger Norton et al**
**Brewer & Son 5-9, Quality Court Chancery Lane**
**London WC2A 1HT(GB)**

## (54) Turning on of cytotoxicity.

(57) The invention provides a method of turning on the cytolytic effector function of human cytolytic T-cells or human natural killer cells, the method involving contacting the cells with a substance or mixture of substances characterized in that the substance or the mixtures binds specifically to the T11 sheep erythrocyte binding glycoprotein of the cells and is capable upon the binding of turning on the cytolytic effector function. Also provided is a cDNA sequence encoding human T11 or a fragment thereof which is capable of inhibiting T-lymphocyte activation.

EP 0 260 880 A2

# TURNING ON OF CYTOTOXICITY

## Background of the Invention

This invention relates to cytolytic human T-lymphocytes ("T-cells") and human natural killer cells ("NK cells").

The T11 sheep erythrocyte binding glycoprotein [relative molecular mass ($M_r$) 50,000] is expressed throughout human T-lymphocyte ontogeny and appears to play an important physiological role in T-cell activation. The treatment of T cells with certain monoclonal anti-T11 antibodies results in antigen independent polyclonal T-cell activation as assessed by proliferation and lymphokine secretion. In addition, the majority of thymocytes that have not yet acquired the T3-Ti antigen/major histocompatibility complex (MHC) receptor can be activated to express interleukin-2 (IL-2) receptors through this T11 structure.

Three distinct epitopes on the 50K T11 molecule have been identified. While the $T11_1$ and $T11_2$ epitopes are expressed on resting as well as activated T cells, anti-$T11_3$ antibodies recognize a spatially distinct epitope that is preferentially expressed on mitogen-or antigen-activated T lymphocytes and thymocytes. The $T11_3$ epitope appears to represent a conformational change since it can be induced on resting T cells within 30 min at 0°C by treatment with anti-$T11_2$. These studies have also shown that the combination of anti-$T11_2$ and anti-$T11_3$ (and to a lesser extent certain anti-$T11_1$ and anti-$T11_3$ antibodies) can induce IL-1-independent polyclonal T-cell activation as measured by proliferation and lymphokine secretion.

## Summary of the Invention

We have discovered that the cytolytic effector function (i.e., the ability to kill other cells) of human cytolytic T-cells ("$T_c$-cells") and human NK cells can be turned on by contacting the $T_c$ or NK cells with a substance or mixture of substances which binds specifically to the T11 sheep erythrocyte binding glycoprotein of those cells and is capable of turning on the cytolytic effector of those cells. (By "binds specifically" is meant binding to the T11 molecule to the substantial exclusion of other surface molecules.)

In some preferred embodiments, the mixture of substances specifically binding to T11 includes an antibody (preferably monoclonal) specific for the $T11_3$ epitope, together with either an antibody (preferably monoclonal) specific for the $T11_1$ epitope or an antibody (preferably monoclonal) specific for the $T11_2$ epitope (anti-$T11_2$ is preferred over anti-$T11_1$).

The invention permits the extracorporeal turning on of the cytotoxicity of cells of a patient (e.g., leukemia and other cancer patients whose cancer cells are to be killed, or an allograft recipient, whose rejection-inducing T-cells, or whose graft-versus-host disease T-cells are to be killed) so that, when the turned-on cells are reintroduced into the patient, they will attack and kill the patient's deleterious cells. The method of the invention is rapid and effective, and can be used to turn on cytotoxicity of any cells bearing the T11 receptor.

We have also succeeded in producing a cDNA sequence encoding the human T11 molecule. In addition, we have completely sequenced the T11 cDNA, and thus know the deduced amino acid sequence of the T11 protein. We have inserted the T11 cDNA into an expression vector, which was used to transfect mammalian cells, which have been shown to express immunologically functional T11.

The deduced amino acid sequence of T11 has allowed us to identify the functional domains of the T11 molecule, which include the signal peptide, the external domain, the transmembrane anchor, and the internal cytoplasmic domain. Because it is the external domain which is recognized by and binds to the natural ligand of T11 found on human lymphocytes, truncated T11 can be produced by recombinant cells according to the invention, which truncated T11 will be secreted by transfected cells for isolation and use in therapeutic applications which depend on the ability of the external domain of T11 to bind to its ligand, as will be explained in more detail below. The preferred genetic constructions thus are those encoding a human lymphocyte activation inhibiting fragment of T11, from which there is deleted the transmembrane anchor and the cytoplasmic domain. Such fragments generally will bind to human lymphocytes and human red blood cells which express a homologous set of surface structures. A human lymphocyte inhibiting fragment is, as defined herein, a fragment which is capable of competing with the naturally-present T11 on the surface of a human lymphocyte, thus interfering with the ability of the lymphocyte to make contact with its target cells (if the lymphocyte is a cytolytic cell), or with macrophages having T11 binding structures

which permit the cell-to-cell contact necessary for lymphocyte proliferation. To test a fragment for the ability to inhibit lymphocyte proliferation, or the cytotoxic effector function, the fragment is contacted with the lymphocytes prior to stimulation with mitogen, and degree of proliferation is measured, using standard techniques, and the result compared to a control in which the fragment was not used.

Other features and advantages of the invention will be apparent from the following description of the preferred embodiments thereof, and from the claims.

Fig. 1 is a histogram showing lysis of B-lymphoblastoid target cell lines by T-cells treated with various monoclonal antibodies (αT11);

Fig. 2 is a histogram showing the effect of anti-T11 antibodies on the cytolytic effector function of various cell lines;

Fig. 3 is a histogram showing lysis of various target cells by T-cell clones QQ and JT3;

Fig. 4 is a diagrammatic representation of the strategy used in sequencing the cDNA (PB1) encoding T11;

Fig. 5 is the sequence of the PB1 cDNA, with the deduced amino acid sequence of T11 given on the bottom line; and

Fig. 6 is a diagrammatic representation of the T11 molecule, showing functional domains.

Purification and Characterization of T11

$4 \times 10^{10}$ Jurkat cells were washed twice in serum-free medium and lysed for one hour at 4° C in 420 ml in 10mM Tris, pH 8.0 containing 0.15 M NaCl, 1% Triton-X 100, 1 mM iodoacetamide and the following protease inhibitors (Sigma): phenylmethylsulfonylfluoride (1mM), chymostatin (0.5 μg/ml), pepstatin (0.5 μg/ml), antipain (0.5 μg/ml), leupeptin (0.5 μg/ml), trypsin inhibitor (0.02 μg/ml). The crude lysate was centrifuged at 3000 × g for 20 min. The supernatant was made 0.5% in sodium deoxycholate and ultra-centrifuged for 60 min at 150,000 × g. $25 \times 10^6$ Jurkat cells from the same culture were surface radiolabelled by lactoperoxidase-catalyzed iodination. $2 \times 10^7$ radiolabelled cells were treated with 0.5 ml of lysis buffer and added to the large scale lysate. The combined lysates were applied at 0.75 ml/min to a 15 ml pre-clear column containing irrelevant mouse monoclonal antibodies anti-T3 (8C8), anti-$Ti_3$ (9H5) and anti-β2 microglobulin coupled to protein A Sepharose beads CL-4B (Pharmacia) at 5 mg antibody per ml of beads, followed by a 5 ml specific antibody column containing anti-$T11_1$ (8B5) coupled to protein A Sepharose at 5 mg/ml. The anti-T11 column was washed with 10mM Tris, pH 8.0 with or without detergents and eluted in 1 ml fractions with 0.1M glycine, PH 3.0, 0.5% Triton-X 100. The fractions were collected in tubes containing 60 μl 1M Tris pH 8.0.

Fractions containing radioactivity were pooled, made 10% in glycerol and 2% in SDS, heated at 60° C for 20 min, loaded onto a 10% preparative polyacrylamide gel in a single lane 10 cm wide, and electrophoresed for 16 h at 40 volts. A 0.5 cm wide strip of the gel was dried and autoradiographed and the rest stained with Coomassie blue. Stained bands containing T11 were localized by comparison with the migration of surface labelled T11 as shown by the autoradiographed strip. Three regions of the gel at approximately 55, 53 and 50KD were excised. Gel slices were washed with $H_2O$ and electroeluted in 50mM ammonium bicarbonate containing 0.1% SDS for 16 h at 50 volts (Hunkapiller et al., 1983). Sample eluates were collected and proteins precipitated at -20° C for 16 h by the addition of 9 volumes of cold ethanol. Precipitated proteins were collected by centrifugation and the protein pellets vacuum dried.

The ethanol precipitates were resuspended in 0.1% SDS and proteins were sequenced by Edman degradation on a gas phase protein sequenator (Applied Biosystems, model 120A) using the 03RPTH program and aqueous trifluoroacetic acid conversion chemistry. The PTH amino acids were identified with an on-line PTH analyzer (Applied Biosystems, model 120A) using a narrow-bore C18 reverse phase column run in acetate buffered 5% aqueous tetrahydrofuran and developed with acetonitrile. 5-10 μg from the 55, 53 and 50 KD bands were analyzed and an identical N-terminal sequence was obtained for each. 18 of the 19 N-terminal positions were assigned as follows:

```
1                 10                18
K E I T N A L E T XXX G A L G Q D I N
```

Turning on of Cytotoxicity

To determine whether T11 triggering could activate $T_c$-cell effector function, we examined the effect of anti-T11 monoclonal antibodies on the lysis of target cells by alloreactive $T_c$-cell clones (Fig. 1). QQ is a representative $T8^+$ $T_c$-cell clone specific for the HLA-B7 molecule expressed by Laz 156, an allogeneic Epstein-Barr virus (EBV)-transformed B-lymphoblastoid cell line with the HLA phenotype A2, A3, B7, B40, Dr2, 4.

Peripheral blood mononuclear cells isolated by Ficoll-Hypaque density gradient centrifugation were stimulated with irradiated (5,000 R) Laz 156 and cloned on day 5 in soft agar. Clones were expanded in liquid culture by restimulation with irradiated Laz 156 and IL-2-containing supernatants. The surface phenotype was determined by indirect immunofluorescence on an Epics V cell sorter. Laz 509 is an EBV-transformed B-cell line from an HLA-disparate donor (HLA genotype: A2, A25, B13, Bw38, Cw6, Dr7). The monoclonal antibodies anti-$T11_{1c}$ (IgG2b), anti-$T11_2$(IgG2a) and anti-$T11_3$(IgG3) were produced as described in Meuer et al. (1984) Cell 36, 897, and used in ascites form at a 1:100 final dilution. Lysis was measured using a standard Cr-release assay in which $T_c$ cells and labelled targets were mixed at a 3:1 ratio in the presence of the indicated concentrations of antibody in a final volume of 0.2 ml. After centrifugation (1,200 g, 10 min), assay plates were incubated for 4h at 37°C before re-centrifugation and removal of aliquots of supernatant for assay of $\gamma$ radioactivity.

As shown in Fig. 1, in the absence of anti-TII antibodies QQ cells lyse Laz 156 but not Laz 509. Anti-$TII_1$ strongly inhibits this specific lysis of Laz 156. Similar inhibitory effects have been observed with other anti-T11 antibodies that block rosetting (Martin et al., 1983, 131 J. Immun. 330). However, saturating concentrations of anti-$T11_2$ or anti-$T11_3$ give only minimal inhibition of the specific cytolysis. More importantly, anti-$T11_3$ in combination with either anti-$T11_1$ or anti-$T11_2$ induces clone QQ to lyse the inappropriate lymphoblastoid target Laz 509. Monoclonal antibodies to other cell-surface markers expressed by QQ (anti-T1) or not expressed by QQ (anti-T4 and anti-T6) separately or together have no such effect. Thus, anti-T11-induced cytotoxicty results from the synergistic effects of particular combinations of anti-T11 monoclonal antibodies (anti-$T11_1$ plus anti-$T11_3$ or anti-$T11_2$ plus anti-$T11_3$).

This result suggests that cross-linking of T11 may be important in T-cell activation, although other factors appear to be involved because the combination of anti-$T11_1$ and anti-$T11_2$ does not induce activation and because previous studies have shown that anti-$T11_2$ or anti-$T11_3$ alone cannot induce T-cell proliferation when coupled to Sepharose.

The induction of antigen-independent cytoxicity by anti-T11 antibodies clearly results from the action of the antibodies on the T-cell clone because (1) the target cells do not express T11 and (2) the anti-T11 antibodies do not induce lysis of target cells in the absence of T-cells. Furthermore, the effect can also be observed if the T-cells are pretreated with the anti-T11 antibodies.

Referring to Fig. 2, various cell lines were either treated (b) or not treated (a) with anti-$T11_2$ and anti-$T11_3$. The two other $T_c$-cell clones, HH and J, were induced to express nonspecific cytolytic activity by treatment with anti-$T11_2$ plus anti-$T11_3$. In contrast, the two alloreactive non-cytotoxic inducer clones, AA7 and AA10, were not triggered to kill by treatment with anti-$T11_2$ and anti-$T11_3$ antibodies. Thus, it appears that T lymphocytes that have acquired cytolytic function during differentiation can be induced to lyse a variety of target cells in an apparently nonspecific fashion by treatment with appropriate combination of anti-T11 antibodies. On the other hand, cytotoxicity is not induced in clones whose active genetic programme lacks the cytotoxic machinery.

Referring to Table 1, below, in order to determine whether anti-T11-induced cytotoxicity occurs through effector mechanisms analogous to those involved in specific cytotoxicity, we compared antigen-specific and anti-T11-induced cytotoxicity with respect to parameters affecting cytolytic activity including calcium requirements, temperature sensitivity, cell-contact dependence and susceptibility to inhibition by anti-T8 antibodies.

Table 1

| Cytolytic T-cell | Conditions | Anti-T11₂+ Anti-T11₃ | %Specific ⁵¹Cr release | |
|---|---|---|---|---|
| | | | Laz 509 | Laz 156 |
| QQ | Control | - | 6 | 81 |
| QQ | Control | + | 47 | 68 |
| QQ | 5 mM EGTA | - | 0 | 1 |
| QQ | 5 mM EGTA | + | 2 | 7 |
| QQ | 25°C | - | 2 | 42 |
| QQ | 25°C | + | 3 | 5 |
| None | Supernatant* | - | 1 | 0 |
| QQ | Anti-T8 | - | 6 | 9 |
| QQ | Anti-T8 | + | 50 | 54 |

*Supernatant of clone QQ triggered with anti-$T11_2$ and anti-$T11_3$ at final dilutions of 1:00 each for 4 h.

Referring still to Table 1, the lysis of Laz 509 and Laz 156 by T-cell clone QQ was measured in the absence and presence of anti-T11 antibodies in the indicated conditions. Anti-T11 antibodies were used in ascites form at a 1:100 final dilution. In one set of wells, QQ cells were omitted and replaced with cell-free supernatants of clone QQ triggered with anti-$T11_2$ and anti-$T11_3$ for 4 h. The supernatants were used at a 50% final concentration.

Calcium is known to be required in the lethal hit stage of antigen-specific cytotoxicity. We found that it is also required for anti-T11-induced cytotoxicity because lysis is almost completely inhibited by EGTA. In addition, both antigen-specific and anti-T11-induced cytotoxicity are inhibited to some extent by low temperature; anti-T11 antibodies are almost completely inhibited at 25°C, whilst specific killing of Laz 156 by QQ is reduced by ~50%. No stable cytolytic factor was detected in the supernatants of T-cell clones triggered with anti-$T11_2$ plus anti-$T11_3$ in this assay system. Consequently, it is likely that either cell contact or close effector-target cell proximity is required for anti-T11-induced cytotoxicity.

These mechanistic similarities between antigen-specific and anti-T11-induced killing suggest that T-cells damage target cells in the same way whether activated through the T3 antigen receptor pathway or via the T11 molecule. Although T11 triggering induces lymphotoxin (LT) secretion by T-cell clones, it is unlikely that anti-T11-induced cytotoxicity is mediated by LT alone because the anti-T11-induced lytic effect can be measured in a 4-h assay using a variety of target cells, including those which are resistant to the effects of LT. In contrast, the detection of LT-mediated cytotoxicity generally requires sensitive target cell lines, longer assays and prior target cell conditioning.

The vast majority of human Natural killer (NK) cells, like early thymocytes, lack Ti $\alpha$-chain messenger RNA, and consequently express no surface T3-Ti complex. On the other hand, the 50 kD T11 glycoprotein is found on the surface of most NK cells. These results suggest that certain NK cells may be related to T-lineage precursors.

Referring to Fig. 3, various target cells were incubated with $T_c$ and NK clones in the absence (a) or presence (b) of anti-$T11_2$ and anti-$T11_3$. In the absence of monoclonal antibodies, the representative $T11^+T3^-$NK clone JT3 efficiently kills the NK-sensitive target cell K562 but not peripheral blood lymphocytes of either of the two B-lymphoblastoid target lines tested. This specificity pattern is clearly distinct from that of the Laz 156-specific T-cell clone QQ. However, after incubation with anti-$T11_2$ and anti-$T11_3$ antibodies, both JT3 and QQ kill all of the target cells to varying degrees. Similar data were obtained for the independently derived NK cell clone $JT_{B18}$ (Hercend et al., 1983, 301 Nature 158). This ability of anti-T11 antibodies to induce killing activity from cells that lack a T3-Ti antigen/MHC receptor complex suggests that the T11 molecule represents an alternative pathway of cell activation. More importantly, these findings suggest that the T11 molecule is a critical structure for inducing cytolytic function in NK cells as well as T-cells.

The ability of anti-$T11_1$ antibodies to inhibit antigen-specific cytolysis suggests that the T11 molecule may also be essential for the activation of cytolytic effector function through the T3-Ti complex. This notion is supported by the observation that the combination of anti-$T11_2$ plus anti-$T11_3$ induces nonspecific cytotoxicity and yet reduces the level of specific killing to that of nonspecific killing (Fig. 1).

## Isolation of cDNA Clones

A cDNA library was constructed as follows. RNA was isolated from the T4 helper clone 5B by the method of Chirgwin et al. (1979). Polyadenylated RNA was isolated by oligo(dT)-cellulose chromatography (Pharmacia PL type 7). cDNA was cloned into the expression vector pCDVI (Okayama & Berg, 1983). The vector was purchased from Pharmacia and the library constructed according to the manufacturer's protocol.

The 5B library was plated at 5-7 $\times$ $10^3$ colonies per 150 mm plate. Replicas were made onto GeneScreen Plus (NEN), amplified with chloramphenicol at 150 mug/ml, denatured, neutralized, washed, dried, and hybridized with redundant oligonucleotide probes. The above amino acid sequence information was used to design these anti-sense oligonucleotide probes:

```
3'-TTT-CTT-TAA-TGA-TTA-CGA-AA-5'        3'-CCA-GTT-CTA-TAA-TT-5'
     C   C   G   G   G   G  G                G   C   G   G
             T   T       T                   T           T
                 C       C                   C
```

Since a fully redundant set of 20mers matching the first seven amino acids would contain 792 different sequences, we synthesized four independent pools, each containing a redundancy of 196. A 48 redundant 14mer corresponding to the N-terminal residues 14-18 was also synthesized. Probes were synthesized using cyanoethyl phosphoramidites on an Applied Biosystems model 381A DNA synthesizer. After cleavage from the controlled pore glass column with $NH_4OH$, they were purified by passage over Sep-Pak C18 column (Millipore) and electrophoresis in 20% polyacrylamide gels.

Purified redundant oligonucleotide probes were end-labelled with T4 polynucleotide kinase (Bethesda Research Labs) using 200 m$\mu$Ci $\alpha$-$^{32}$P-ATP (Amersham) for 0.2 $\mu$g of oligonucleotide. Amplified filters were hybridized with 192 redundant 20mers at 40 C in plaque screen buffer (50mM Tris, pH 7.5, 1 M NaCl, 1% SDS, 0.1 sodium pyrophosphate, 0.2% BSA, 0.2% Ficoll 400, 0.2% PYP) for 16 h and washed at 43 C in plaque screen salt (50mM Tris PH 7.5, 1 M NaCl, 1% SDS, 0.2% sodium pyrophosphate) for 30 min. Colonies positive in the first round of screening were picked, replica plated, and rescreened with the 20mer. Identical replicas were also hybridized with the 48-redundant 14mer at 28C and washed at 31C. The 20mers were used to screen the cDNA library. One pool of 20mers with a redundancy of 196 which hybridized in Northern analysis to T cell specific mRNAs identified 32 clones. These were isolated and rescreened with the 14mer. Seventeen of 32 clones contained sequences complementary to both probes. BamH I digests of plasmid DNA from these clones revealed that 11 contained 1.6Kb inserts, three contained 1.3Kb inserts, and that three clones had lost a BamH I restriction site. A representative 1.6Kb insert from a clone termed PB1 and a representative 1.3Kb insert from a clone termed PB2 were subcloned into the M13 vector mp18 (New England Biolabs) for sequencing by the dideoxy chain termination method. Primers were either the universal primers supplied by the manufacturer or 17mers synthesized on an Applied Biosystems model 381A.

10 $\mu$g of 5B RNA and polyA+ RNA from many cell types were electrophoresed under denaturing conditions in 7% formaldehyde and 0.02M $NaPO_4$, pH 7.0. The RNA was transferred to nitrocellulose and hybridized to $^{32}$P-labeled PB1 DNA in 50% formamide, 5X Denhardt's, 5X SSC, 0.1% SDS and 250 m$\mu$g/ml salmon sperm DNA for 12 h at 42 C. The probe was added at 2 $\times$ $10^6$ cpm/ml. The filter was washed in 2 $\times$ SSC, 0.1% SDS at 25 C for 20 min and in 0.1% SSC, 0.1% SDS at 50 C for 30 min and exposed to film for 48 h with an intensifying screen. In these experiments, PB1 DNA was labelled by random priming as described (Feinberg et al., 1983).

DNA from REX was restriction digested and electrophoresed in a 0.8% agarose gel. The gel was denatured, neutralized and transferred to nitrocellulose using 10 X SSC. Hybridization and washing procedures were as for northern analysis.

Referring to Fig. 5, the 1.6Kb insert of PB1 (solid line) was separated from the plasmid by BamHI digestion and subcloned into the M13 sequencing vector mp18. The M13 universal primer was used to derive initial sequence at the 5′ end. Subsequently, primers of 17 nucleotides were used for dideoxy sequencing. Sequencing reactions were also performed on M13 clones which contained BamH I-Sac I and Sac I-BamH I fragments as shown. The open reading frame of the insert is identified by the thick solid bar.

Referring to Fig. 6, there is shown the cDNA and predicted protein sequence of PB1. The probable signal peptide (———), the $NH_2$-terminus of the mature protein ( ⌊——→ ) and the position of a CNBr cleavage derived fragment ( ⌊══⇒ ) are shown. Polyadenylation signals at nucleotides 1103 and 1505 are underlined. The last nucleotide before the poly(A) tail in clone PB2 is indicated by an arrowhead at position 1125.

The complete nucleotide sequence of clone PB1 is 1,522 bases in length and flanked by a poly(A) sequence at its 3′ end. An open reading frame of 1,080 bases (positions 24-1103) begins with an ATG methionine codon and is flanked by 23 nucleotides of 5′ untranslated sequence and by 419 bases of 3′ untranslated sequence. A polyadenylation signal (AATAAA) is located 18 bases upstream from the beginning of the poly(A) tail (Fig. 6 ). PB2 is identical to PB1 except that it lacks four nucleotides present at the 5′ end of PB1 and has a shorter 3′ untranslated region. A poly(A) tail was noted in PB2 after the nucleotide corresponding to residue 1125 in PB1 (arrow head). The bases from positions 1102 to 1103 form part of a Gln codon at amino acid 336 and a stop codon as well as the first five bases of the polyadenylation signal AATAAA (nucleotides 1102-1106) for mRNA corresponding to clone PB2.

The N-terminal lysine of the mature polypeptide is preceded by a sequence coding for a stretch of 24 hydrophobic amino acids which likely represents the signal sequence required for the T11 precursor to be transported across the endoplasmic reticulum. The cDNA sequence predicts three potential N-linked glycosylation sites (Asn-X-Ser/Thr) on the mature protein at amino acid positions 65, 117 and 126. An extremely hydrophobic stretch of 25 amino acids characteristic in size and composition of a transmembrane domain is found at positions 186-210. This region is followed by seven basic amino acids within the next ten residues consistent with the notion that this is the start of the intracytoplasmic domain. 21% of residues in the region of amino acids 211-336 are prolines. This cDNA sequence predicts a molecular weight for the mature polypeptide backbone of 37,994 daltons.

To determine the molecular weight of the broad 50-55KD band in the absence of N-linked sugars, surface labelled T11 was digested with endoF and analyzed by SDS-PAGE. After digestion with endoF, there is a loss of the 55KD band and the appearance of a major component at approximately 40KD. These data suggest that the 55KD protein exists as a 40KD structure in the absence of any N-linked sugar moieties and is consistent with the above determined molecular weight of the protein. These results are consistent with the possibility that all three N-linked glycosylation sites may be utilized.

Two related T11 mRNA species

Northern analysis using polyA+ RNA or cytoplasmic RNA from a variety of sources indicates that expression of the PB1 and PB2 sequences is T lineage specific and yields two common bands of 1.7 and 1.3Kb. Thus, each of six human T lineage cells tested including activated T helper clones, thymus derived tumors, normal thymocytes, activated T cells, and resting peripheral blood T lymphocytes expresses the 1.7 and 1.3Kb transcripts. In contrast, non-T lineage cells such as normal peripheral blood B cells and macrophages, an EBV-transformed B lymphoblastoid line, Laz 509, and the non-lymphoid hematopoietic cell lines HL-60 and U937 lack both transcripts. Given the similarity in size differences between the PB1 and PB2 inserts (~400 bases) and the two species of mRNA, it is likely that PB1 and PB2 cDNAs correspond to the 1.7Kb and 1.3Kb transcripts, respectively. This is further supported by the finding that an oligonucleotide based on a sequence from the 3′ untranslated region unique to PB1 selectively hybridizes in northern analysis to sequences in the 1.7Kb site region.

Considerable variability exists within the level of expression of the 1.7 and 1.3Kb transcripts among individual T lineage tumor cells and physiologic T cell populations. Also, activated T cells express >10 fold higher amounts than resting T lymphocytes. This result is consistent with data indicating that T11 surface expression increases from 20,000 to 200,000 copies per cell upon activation with mitogens or antigens during a six day period and suggests that this differential expression is at least in part transcriptionally regulated.

PB1 homologous sequences are also expressed in murine thymocytes. However, under the conditions of hybridization, only the 1.3Kb molecular weight species is detected in polyA+ mRNA from Balb/cJ thymocytes. It is thus likely that the murine species lacks the second polyadenylation signal in the 3' untranslated region of a homologous gene. This result also suggests that a highly homologous T11-related structure exists on murine T lineage cells.

The T11 molecule is encoded by a single copy gene

To determine the number of T11 genes present in the human genome, southern analysis was performed with the $^{32}P$-labeled PB1 insert. The probe hybridizes to a set of bonds of approximately 8 and 7Kb in a BamH I digest of genomic REX T cell DNA, to a single band >14Kb in EcoR I restricted REX DNA, and to a set of 7.4, 6.0 and 0.7Kb fragments in Hind III digested REX DNA. Analysis of DNA from granuloctyes gave an identical pattern, indicating that T11 is in the human genome as a single copy gene and, unlike the T cell receptor alpha, beta and gamma genes or the B cell IgH and light chain genes, does not rearrange in lymphoid cells.

PB1 and PB2 cDNAs encode a functional T11 protein

To further prove that the sequences contained in the PB1 and PB2 cDNA clones encode the T11 protein and are themselves necessary and sufficient for T11 surface expression, COS-1 cells were transfected with PB1 or PB2 plasmid cDNAs or the unrelated IL-3 cDNA. COS-1 cells were plated at 4 $\times$ $10^4$ cells/cm$^2$ in 6 well plates in RPMI 1640 (Gibco), 10% fetal calf serum, 0.03% glutamine, 1% penicillin-streptomycin. Six ug of PB2 or IL-3 (a gift of Ken-ichi Arai, DNAX) plasmid DNA was added to 350 µl RPMI and vortexed. 350 µl of 800 µg/ml DEAE-Dextran in RPMI, 100mM Tris, pH 7.4 was added to the plasmid solution and vortexed. This solution was added to one well of cells which were then incubated for 3 h in a 37 C incubator. The medium was aspirated and the cells washed once with RPMI. Subsequently, 2.5 ml of RPMI containing 2% fetal calf serum, 0.03% glutamine, 1% penicillin streptomycin, 150µM chloroquine was added to each well and the cells incubated at 37 C for 3 h. The cells were washed twice with RPMI and after addition of fresh medium, incubated for 48 h at 33 C in a humid atmosphere to assay for surface T11 expression. 10,000 cells in each transfection group were analyzed by fluorescence activated cell sorting.

After 48-72 h, expression of surface T11 on the transfected cell was determined on the basis of anti-T11 monoclonal antibody reactivity measured by indirect immunofluorescence on an Epics V cell sorter and rosette formation with sheep erythrocytes.

The non-transfected COS cells failed to interact with anti-$T11_1$ antibody. In contrast, after transfection with PB2, >25% of COS-1 cells express readily detectable surface T11 molecules. An identical level of reactivity was also detected with an anti-$T11_2$ monoclonal antibody. Similar levels of anti-$T11_1$ and anti-$T11_2$ reactivity resulted from transfections using PB1.

To next determine whether the transfected and expressed PB1 and PB2 sequences could themselves endow COS-1 cells with the ability to form aggregates with SRBC, transfected cells were tested for SRBC binding capacity. Incubation of SRBC with COS-1 cells resulted in no rosette formation. In contrast, after transfection of COS-1 cells with PB2 SRBC rosetting was observed. The SRBC rosette forming capacity was detected in COS-1 cells transfected with either of the two PB cDNAs separately or together. While the SRBC rosette capacity was not inhibited by monoclonal antibodies directed against the T3 subunits of the T3-Ti antigen/MHC receptor complex, the anti-$T11_1$ monoclonal antibody completely abrogated E rosette formation. Collectively, the above data provide unequivocal proof that the PB1 and PB2 cDNA sequences each independently encode a functional T11 protein.

Functional Domains of T11

Referring to Fig.7 , the T11 molecule has a hydrophobic signal peptide (L), which is cleaved upon biosynthesis. The hydrophilic, 185 amino acid long external domain of the molecule is located outside the cell membrane. The hydrophobic 25 amino acid long transmembrane (TM) anchor region is embedded in the T-cell membrane, and the 126 amino acid long cytoplasmic domain of the molecule remains in the cytosol. In Fig. 3, the proline-rich region of this domain is indicated by P-P-P. The amino acid numbering corresponds to the mature peptide.

Cells transfected with PB1, like T-cells which naturally produce T11, do not secrete T11, but rather retain the T11 molecule by means of the transmembrane anchor, with only the external domain exposed. A truncated T11 molecule, with the transmembrane and cytoplasmic domains deleted, can, unlike the complete T11 molecule, be secreted by transfected cells and used in the applications described below. Such a truncated molecule can be prepared in a manner analogous to the method by which an anchor-minus IL-2 receptor molecule was prepared by Treiger et al. (1986) J. Immunol. 136, 4099. The truncated IL-2 receptor was found to be capable of binding to its ligand, IL-2.

To make the truncated T11 molecule, PB1 or PB2 cDNA representing the gene for the entire T11 molecule will be restricted with Pvu II. This enzyme uniquely cuts within the 1,522 base pair T11 molecule cDNA insert at base 629, resulting in removal of all transmembrane and intracytoplasmic sequences and seven amino acids of the external domain. Subsequently, a 14 base phosphorylated synthetic oligonucleotide (CTAAGAATTCTTAG) containing the third base of the codon for amino acid 178, a termination codon TAA followed by the six base recognition sequence for EcoR I (GAATTC), and four nucleotides (TTAG) complementary to CTAA, will be ligated to the Pvu II restricted plasmid DNA with T4 DNA ligase. The DNA will then be digested with Pst I to separate the 5′ end of the cDNA insert from the plasmid DNA and then subcloned into an appropriate expression vector. For example, this fragment could be blunted by T4 DNA polymerase to remove the Pst site and then be ligated to the EcoR I linker by T4 ligase. Finally, it could be digested with EcoR I before ligation into the unique EcoR I site of the publicly available PcEXV-1 expression vector.

## Use

Human NK and $T_c$ cells can be activated via the T11 molecule in vitro or in vivo and used in the treatment of any medical condition characterized by the presence of unwanted cells. In particular, the method can be used to turn on the cytotoxicity of $T_c$ and NK cells so that they will attack and kill pathogen-infected cells, e.g., cells infected with bacterial, fungal, viral, or protozoan pathogens; or tumor cells, e.g., lung, colorectal, or esophageal cancers.

The first step in in vitro activation is to obtain resting NK and/or $T_c$ cells, either from the patient or a suitable donor. This is typically done by separating out lymphocytes from blood and then, optionally, culturing the lymphocytes in the presence of Interleukin-2 to expand their numbers. (The methods by which lymphocytes are separated out, cultured, treated with Interleukin-2, and used to treat cancer patients are described in detail in Rosenberg et al. (1985) New Eng. J. Med. 313 , 1485.)

The lymphocytes and/or NK cells are incubated with the cytotoxicity-inducing substance for a relatively short time period (e.g., four hours at 30-40°C, in the presence of calcium ions), and subsequently infused into the patient. Infusion can be via an arterial or venous catheter or into a large peripheral vein.

Alternatively, rather than activating $T_c$ and NK cells in vitro, the activating substance can be administered in vivo, most preferably by direct perfusion of the tumor with the substance, e.g., via the hepatic artery to induce cytotoxicity of existing NK and $T_c$ Cells.

The methods of the invention induces cytotoxicity in a way which bypasses the normal T11 recognition mechanism, and enables the resultant activated NK and $T_c$ cells to attack and kill their target cells without further treatment. (NK cells have a broad range of target specificity, while $T_c$ cells recognize tumor cells by virtue of tumor-specific antigens.) Unlike treatment with IL-2 alone, treatment according to the invention will activate the totality of NK and $T_c$ cells, and be complete in a few hours.

The T11 protein, or its truncated, secreted form, can be used in a variety of diagnostic and therapeutic applications, all of which are based on the binding of T11 to its natural ligand on human lymphocytes and homologous surface structures present on target cells which facilitate T lymphocyte - target cell interactions, which result in target cell lysis or lymphocyte proliferation. (Siliciano et al. Nature 317:428-431 (1985); and Palacios and Martinez-Maza, J. Immunol. 129:2479-2485 (1982).

The T11 molecule is expressed on the surface of many human T-cell malignancies, e.g., T-cell leukemias and lymphomas. In addition, autoimmune diseases. e.g., rheumatoid arthritis and Systemic Lupus Erythmatosis (SLE), are characterized by the presence in the blood and lymph of large numbers of T11-bearing T-cells. Rapid cell turnover in these disease states can cause the shedding of the T11 molecule into the bloodstream.

T11 can be used as an immunogen to produce polyclonal or monoclonal anti-T11 antibodies, using conventional techniques. These antibodies can be labeled with any conventional label, e.g., radioisotopes, and used in conventional immunoassay methods to measure serum T11 levels and thus monitor patients having T-cell-associated diseases. Particularly sensitive ELISA-type assays will employ two anti-T11 antibodies, each to a different antigenic determinant on the surface of T11, in a sandwich format.

Another use for the anti-T11 antibodies is in the purification of recombinant T11, produced as described above. The anti-T11 is coupled to a column and T11-containing media is passed through the column so that the T11 reversibly binds to the T11, after which T11 is eluted in purified form, according to conventional methods.

The disease states which can be treated using T11 include medical conditions characterized by unwanted activity of the immune system which results in excess T-cell activation, which plays a key role in the amplification of the immune response. These conditions include rheumatoid arthritis; Systemic Lupus Erythmatosis; juvenile onset diabetes; multiple sclerosis; allergic conditions; inflammatory conditions such as eczema, ulcerative colitis, inflammatory bowel disease, and Crohn's disease; and allograft rejection (e.g., rejection of a transplanted heart or kidney). Soluble T11 competes with the surface-bound T11 for its ligand on target cells thus dampening immune response amplification. The T11 admixed with a Pharmaceutically acceptable carrier substance such as saline, is administered intravenously to a human patient in an effective amount, e.g., 20 $\mu$g to 500 $\mu$g per kg body weight. For some conditions, T11 can be administered directly to the site where needed most; for example, T11 can be injected directly into the inflamed joint of a human patient suffering from rhematoid arthritis.

Deposits

Plasmid PB1 was deposited in the American Type Culture Collection, Rockville, Maryland on October 1, 1986 and assigned ATCC Accession Number 40268.

Dana-Farber Cancer Institute, Inc. agrees that the designated culture having ATCC Accession Number 40268 will be maintained throughout the effective life of a patent granted, for 30 years from the date of deposit, or for 5 years after the last request for the deposit after issuance of the patent, whichever is the longer, and it guarantees replenishment of the culture having ATCC Accession Number 40268 at the depository in the event that a deposit becomes nonviable during the effective life of a patent granted, for 30 years from the date of deposit, or for 5 years after the last request for the deposit after issuance of the patent, whichever is longer.

## Claims

1. A method of turning on the cytolytic effector function of human cytolytic T-cells or human natural killer cells, said method comprising contacting said cells with a substance or mixture of substances characterized in that said substance or said mixture binds specifically to the T11 sheep erythrocyte binding glycoprotein of said cells and is capable upon said binding of turning on said cytolytic effector function.

2. A method according to claim 1 wherein said mixture of substances comprises an antibody specific for the $T11_3$ epitope and either an antibody for the $T11_1$ epitope or an antibody for the $T11_2$ epitope.

3. A method according to claim 2 wherein said antibodies are monoclonal antibodies.

4. A method according to any one of the preceding claims wherein said cells are obtained from a human patient and said contacting is carried out extracorporeally.

5. The method of claim 1 wherein said contacting is carried out in a human patient in vivo.

6. A cDNA sequence encoding human T11 or a fragment thereof which is capable of inhibiting T-lymphocyte activation.

7. A vector including a DNA sequence encoding human T11 or a fragment thereof which is capable of inhibiting T-lymphocyte activation.

8. A cDNA sequence according to claim 6 wherein said cDNA sequence encodes a T11 fragment in which the transmemtrane anchor and cytoplasmic domain are deleted.

9. A vector according to claim 7 wherein said DNA sequence encodes a T11 fragment in which the transmembrane anchor and cytoplasmic domain are deleted.

10. Monoclonal antibodies against an antigen comprising a peptide encoded by the cDNA according to claim 6 or a fragment thereof.

11. A substantially purified protein or peptide encoded by the cDNA according to claim 6.

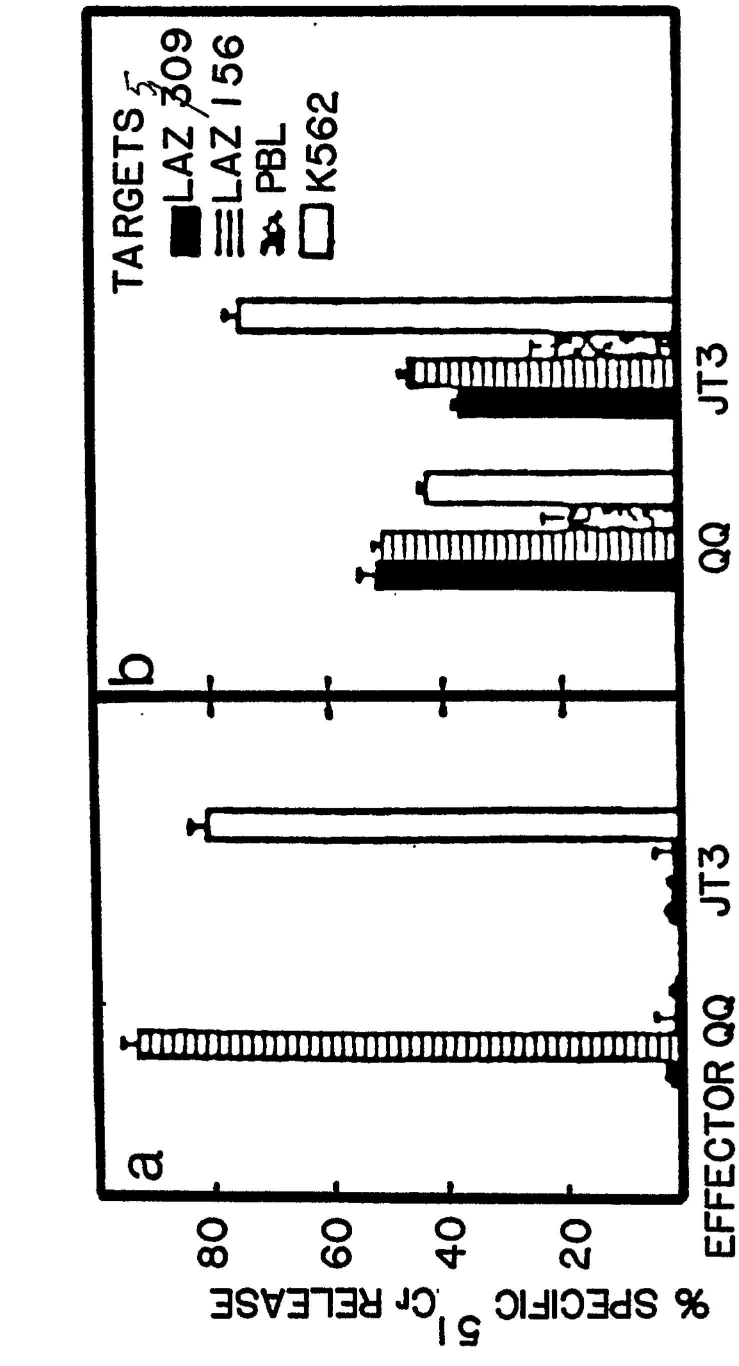
a
b
% SPECIFIC 51Cr RELEASE
80
60
40
20
EFFECTOR QQ
JT3
QQ
JT3
TARGETS
LAZ 509
LAZ 156
PBL
K562

FIG. I

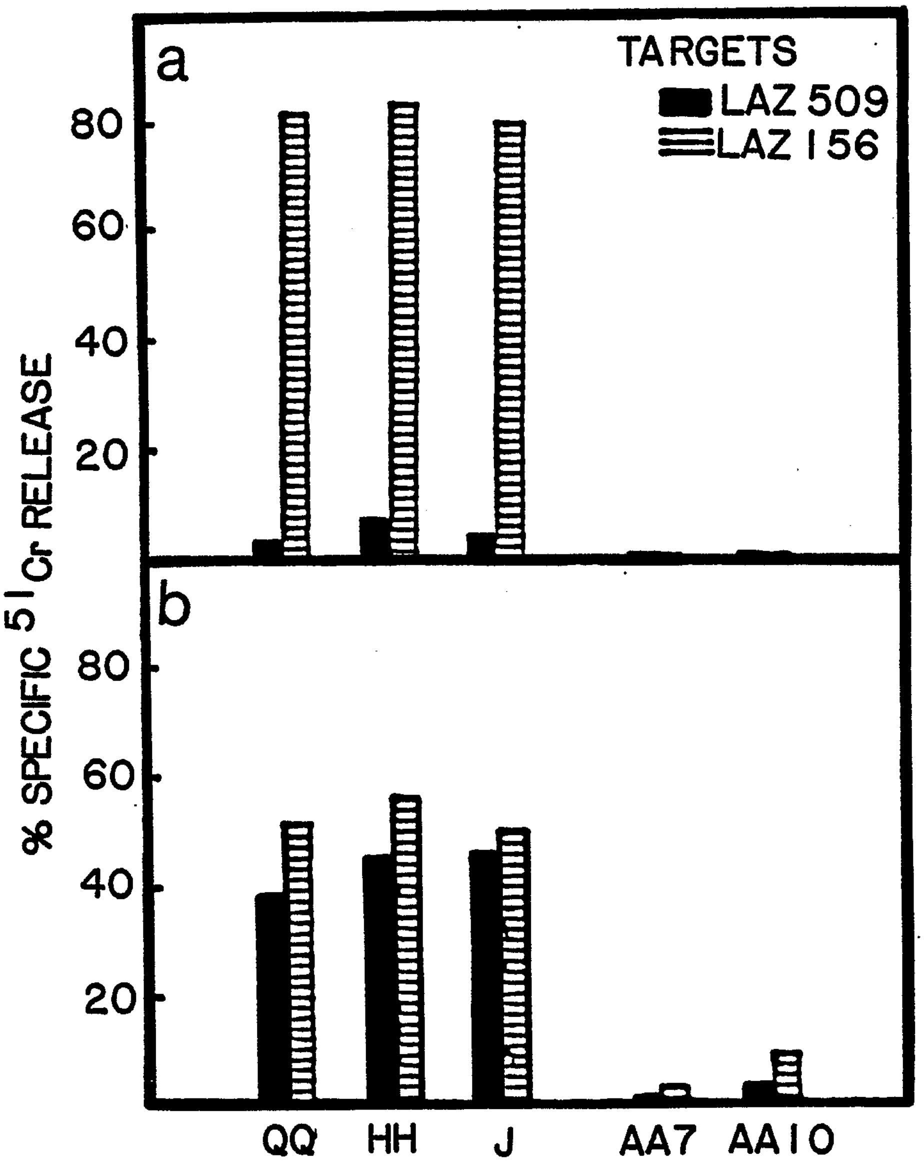

a
TARGETS
LAZ 509
LAZ 156
80
60
40
20
b
80
60
40
20
% SPECIFIC 51Cr RELEASE
QQ
HH
J
AA7
AA10


FIG. 2

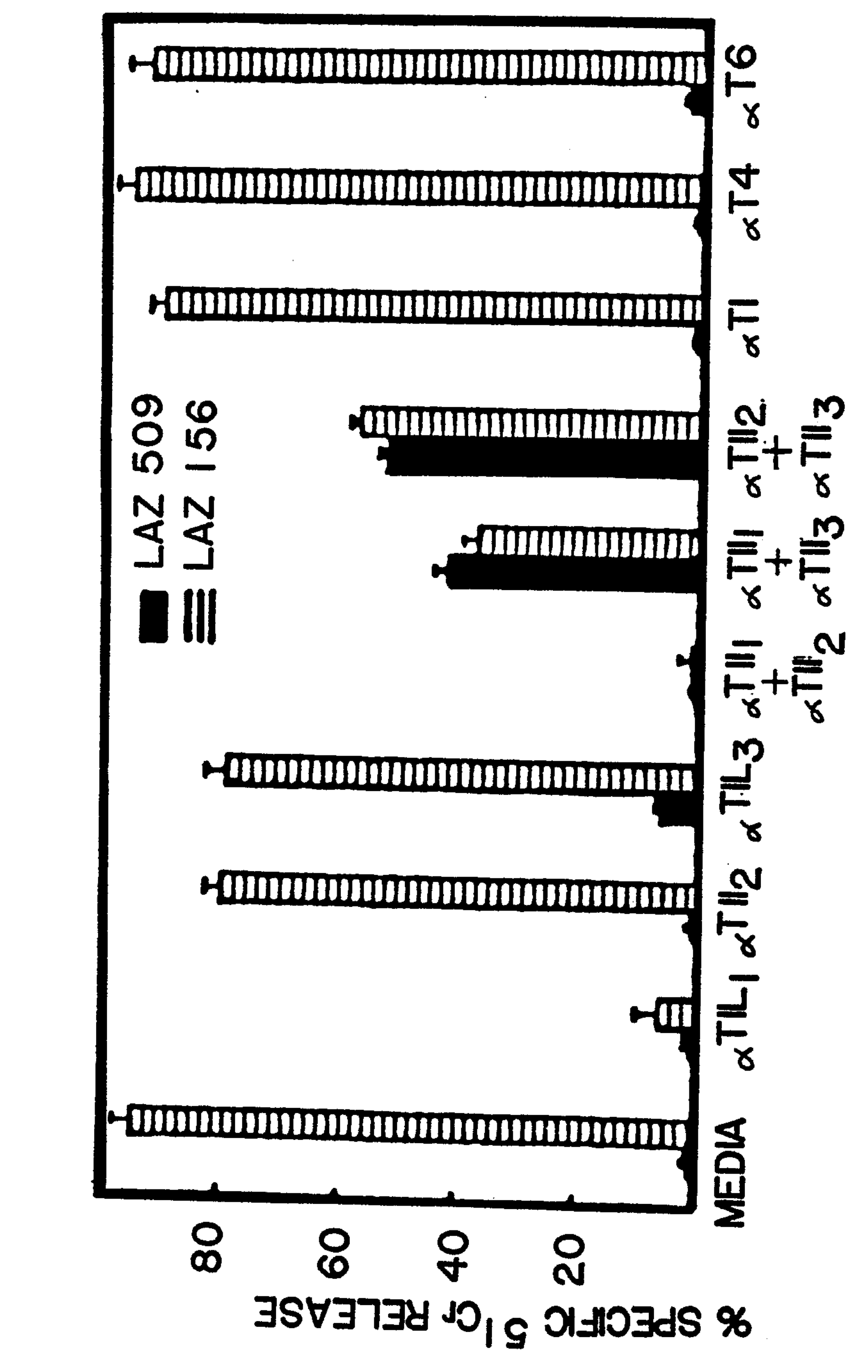
LAZ 509
LAZ 156
% SPECIFIC 51Cr RELEASE
80
60
40
20
MEDIA
αTIL1
αTII2
αTIL3
αTII1 + αTII2
αTII1 + αTII3
αTII2 + αTII3
αTI
αT4
αT6

FIG. 3

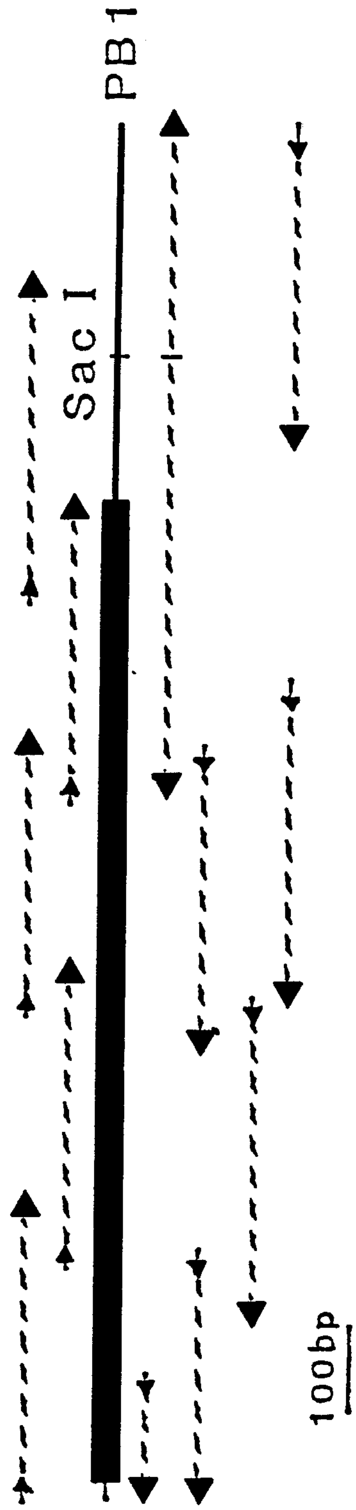


FIG. 4

```
          10         20                         38                      53
AAAACAGGAA ACCAACCCCT AAG ATG AGC TTT CCA TGT AAA TTT GTA GCC AGC TTC
                          MET Ser Phe Pro Cys Lys Phe Val Ala Ser Phe      -14

                68              83              98
CTT CTG ATT TTC AAT GTT TCT TCC AAA GGT GCA GTC TCC AAA GAG ATT ACC AAT
Leu Leu Ile Phe Asn Val Ser Ser Lys Gly Ala Val Ser Lys Glu Ile Thr Asn     5
                                                    ---> N-term
113             128             143             158
GCC TTG GAA ACC TGG GGT GCC TTG GGT CAG GAC ATC AAC TTG GAC ATT CCT AGT
Ala Leu Glu Thr Trp Gly Ala Leu Gly Gln Asp Ile Asn Leu Asp Ile Pro Ser    23

        173             188             203             218
TTT CAA ATG AGT GAT GAT ATT GAC GAT ATA AAA TGG GAA AAA ACT TCA GAC AAG
Phe Gln MET Ser Asp Asp Ile Asp Asp Ile Lys Trp Glu Lys Thr Ser Asp Lys    41

                233             248             263
AAA AAG ATT GCA CAA TTC AGA AAA GAG AAA GAG ACT TTC AAG GAA AAA GAT ACA
Lys Lys Ile Ala Gln Phe Arg Lys Glu Lys Glu Thr Phe Lys Glu Lys Asp Thr    59

    278             293             308             323
TAT AAG CTA TTT AAA AAT GGA ACT CTG AAA ATT AAG CAT CTG AAG ACC GAT GAT
Tyr Lys Leu Phe Lys Asn Gly Thr Leu Lys Ile Lys His Leu Lys Thr Asp Asp    77
```

FIG. 5-1

FIG. 5-2

338 353 368
CAC GAT ATC TAT AAG GTA TCA ATA TAT GAT ACA AAA GGA AAA AAT GTG TTG GAA
Gln Asp Ile Tyr Lys Val Ser Ile Tyr Asp Thr Lys Gly Lys Asn Val Leu Glu 95

383 398 413 428
AAA ATA TTT GAT TTG AAG ATT CAA GAG AGG GTC TCA AAA CCA AAG ATC TCC TGG
Lys Ile Phe Asp Leu Lys Ile Gln Glu Arg Val Ser Lys Pro Lys Ile Ser Trp 113

443 458 473 488
ACT TGT ATC AAC ACA ACC CTG ACC TGT GAG GTA ATG AAT GGA ACT GAC CCC GAA
Thr Cys Ile Asn Thr Thr Leu Thr Cys Glu Val MET Asn Gly Thr Asp Pro Glu 131

CNBr

503 518 533
TTA AAC CTG TAT CAA GAT GGG AAA CAT CTA AAA CTT TCT CAG AGG GTC ATC ACA
Leu Asn Leu Tyr Gln Asp Gly Lys His Leu Lys Leu Ser Gln Arg Val Ile Thr 149

548 563 578 593
CAC AAG TGG ACC ACC AGC CTG AGT GCA AAA TTC AAG TGC ACA GCA GGG AAC AAA
His Lys Trp Thr Thr Ser Leu Ser Ala Lys Phe Lys Cys Thr Ala Gly Asn Lys 167

608 623 638
GTC AGC AAG GAA TCC AGT GTC GAG CCT GTC AGC TGT CCA GAG AAA GGT CTG GAC
Val Ser Lys Glu Ser Ser Val Glu Pro Val Ser Cys Pro Glu Lys Gly Leu Asp 185

653 668 683 698
ATC TAT CTC ATC ATT GGC ATA TGT GGA GGA GGC AGC CTC TTG ATG GTC TTT GTG
Ile Tyr Leu Ile Ile Gly Ile Cys Gly Gly Gly Ser Leu Leu MET Val Phe Val 203

FIG. 5-3

```
    713                         728                         743                         758
GCA CTC CTC GTT TTC TAT ATC ACC AAA AGG AAA AAA CAG AGC AGT CGC ACA AAT
Ala Leu Leu Val Phe Tyr Ile Thr Lys Arg Lys Lys Gln Arg Ser Arg Arg Asn    221

                        773                         788                         803
GAT GAG GAG CTG GAG ACA AGA GCC CAC AGA GTA GCT ACT GAA GAA AGG GGC CGG
Asp Glu Glu Leu Glu Thr Arg Ala His Arg Val Ala Thr Glu Glu Arg Gly Arg    239

    818                         833                         848                         863
AAG CCC CAC CAA ATT CCA GCT TCA ACC CCT CAG AAT CCA GCA ACT TCC CAA CAT
Lys Pro His Gln Ile Pro Ala Ser Thr Pro Gln Asn Pro Ala Thr Ser Gln His    257

            878                         893                         908
CCT CCT CCA CCA CCT GGT CAT CGT TCC CAG GCA CCT AGT CAT CGT CCC CCG CCT
Pro Pro Pro Pro Pro Gly His Arg Ser Gln Ala Pro Ser His Arg Pro Pro Pro    275

923                         938                         953                         968
CCT GGA CAC CGT GTT CAG CAC CAG CCT CAG AAG AGG CCT CCT GCT CCG TCG GGC
Pro Gly His Arg Val Gln His Gln Pro Gln Lys Arg Pro Pro Ala Pro Ser Gly    293

        983                         998                         1013                        1028
ACA CAA GTT CAC CAG CAG AAA GGC CCG CCC CTC CCC AGA CCT CGA GTT CAG CCA
Thr Gln Val His Gln Gln Lys Gly Pro Pro Leu Pro Arg Pro Arg Val Gln Pro    311

                1043                        1058                        1073
AAA CCT CCC ATG GGG CAG CAG AAA ACT CAT TGT CCC CTT CCT CTA ATT AAA AAA
Lys Pro Pro MET Gly Gln Gln Lys Thr His Cys Pro Leu Pro Leu Ile Lys Lys    329
```

```
1088                        1103           1116       1126       1136       1146
GAT AGA AAC TGT CTT TTT CAA TAA AAAGCACTGT GGATTTCTGC CCTCCTCATG TGCATATCCG   336
Asp Arg Asn Cys Leu Phe Gln

      1156       1166       1176       1186       1196       1206       1216
TACTTCCATG AGCTGTTTTC TCTGTGCAGA ACATTGTCAC CTCCTGAGCC TGTGCCCCAC AGCCACCTCT

      1226       1236       1246       1256       1266       1276       1286
GCATCTTCGA ACTCAGCCAT GTGGTCAACA TCTCGAGTTT TTGGTCTCCT CAGAGAGCTC CATCACACCA

      1296       1306       1316       1326       1336       1346       1356
GTAAGGAGAA GCAATATAAG TGTCATTGCA AGAAGTGTAG AGGACCGAGC CAGAAATCTT AGAGATTTCT

      1366       1376       1386       1396       1406       1416       1426
TGTCCCCTCT CAGGTCATGT GTAGATGCGA TAAATCAAGT GATTGGTGTG CCTCGGTCTC ACTACAACCA

      1436       1446       1456       1466       1476       1486       1496
GCCTATCTGC TTAACAGACT CTGGAGTTTC TTATGTGCCC TGGTCGACAC TTGCCCACCA TCCTCTCAGT

      1506       1516
AAAACTGAAA TAAAAGCTTT GACTAGA
```

FIG. 5-4

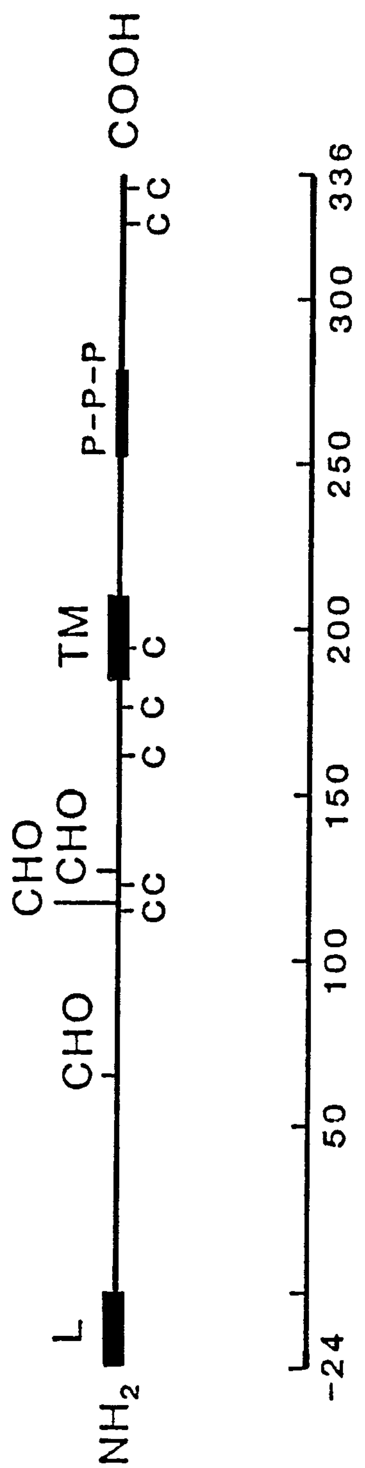

NH$_2$
L
CHO
CHO
CHO
CC
C C
C
TM
P-P-P
C C
COOH
-24
50
100
150
200
250
300
336


FIG. 6